# EUROPÄISCHE PATENTSCHRIFT

(11) **EP 0 973 709 B1**
(45) Veröffentlichungstag und Bekanntmachung des Hinweises auf die Patenterteilung: **20.11.2002**
(21) Anmeldenummer: 98910635.6
(22) Anmeldetag: 09.02.1998
(51) Int. Cl.: C07C 17/38, C07F 7/16, C07C 19/03

(54) **VERFAHREN ZUR REINIGUNG VON MIT C1-C3-KOHLENWASSERSTOFFEN UND/ODER ISO-BUTAN VERUNREINIGTEM METHYLCHLORID**
METHOD FOR CLEANING METHYL CHLORIDE CONTAMINATED WITH C1-C3 HYDROCARBONS AND/OR ISOBUTANE
PROCEDE DE PURIFICATION DE CHLORURE DE METHYLE CONTAMINE PAR DES HYDROCARBURES C1-C3 ET/OU DE L'ISOBUTANE

(30) Priorität: 20.02.1997 DE 19706649
(43) Veröffentlichungstag der Anmeldung: 26.01.2000
(73) Patentinhaber: GE Bayer Silicones GmbH & Co. KG, 40699 Erkrath (DE)
(72) Erfinder: FELDNER, Kurt, D-51375 Leverkusen (DE); WEBER, Rainer, D-51519 Odenthal (DE); HAUSCHILD, Torsten, D-51469 Bergisch Gladbach (DE); MÄCKER, Ralf, D-51379 Leverkusen (DE); RUFFERT, Gerhard, D-51377 Leverkusen (DE)
(86) Internationale Anmeldenummer: EP9800701
(87) Internationale Veröffentlichungsnummer: WO98037044

(56) Entgegenhaltungen:
- CH-A- 253 706
- DE-A- 3 610 706
- US-A- 5 453 113
- DATABASE WPI Section Ch, Week 9023 Derwent Publications Ltd., London, GB; Class E11, AN 90-177372 XP002068390 & SU 1 502 557 A (HETEROORG CPDS AS USSR)

## Beschreibung

Die Erfindung betrifft ein Verfahren zur Herstellung von Methylchlorsilanen durch Umsetzung von Silizium mit Methylchlorid in Anwesenheit von Katalysatoren und ggf. Promotoren, das die Schritte umfasst: Abtrennung der entstandenen Methylchlorsilane in einer Destillationskolonne; Zuführen des in der genannten Destillationskolonne abgetrennten Gasgemisches, das Methylchlorid, C₁-C₃-Kohlenwasserstoffe und Iso-Butan enthält, in eine Destillationskolonne. Insbesondere betrifft die Erfindung ein Verfahren zur Entfernung von Verunreinigungen aus Methylchlorid, das bei der Herstellung von Methylchlorsilanen eingesetzt wird.

Bei der Herstellung von Methylchlorsilanen durch Umsetzung von Silicium mit Methylchlorid (CH₃Cl), wie sie grundlegend in US-A 2 380 995 beschrieben ist. entstehen neben den verschiedenen Methylchlorsilanen auch gesättigte und ungesättigte Kohlenwasserstoffe.

Für die industrielle Durchführung des Verfahrens ist es erforderlich, das nach einmaligem Überleiten über die Kontaktmischung nicht umgesetzte Methylchlorid zurückzugewinnen und erneut einzusetzen.

Nach der destillativen Abtrennung der Methylchlorsilane aus dem den Reaktor verlassenden Gasstrom findet man niedrig siedende Kohlenwasserstoffe, wie Methan, Ethan, Propan, Ethen und Propen, in dem wiedereinzusetzenden Methylchlorid. Dadurch kommt es zu einer Anreicherung dieser Stoffe im Methylchlorid-Kreislauf, die sich negativ auf die Umsetzung von Silicium mit Methylchlorid auswirkt.

Zur Unterdrückung dieser Anreicherung wird das zurückzugewinnende Methylchlorid in der Regel komprimiert und kondensiert, wobei ein Teil der niedrig siedenden Bestandteile wie Methan, Ethan und Ethen entfernt wird.

Bei dieser Verfahrensweise wird Methylchlorid erhalten, das noch Reste an Methan, Ethan und Ethen und als wesentliche Verunreinigungen C₃-Kohlenwasserstoffe wie Propan und Propen sowie iso-Butan enthält. Iso-Butan ist dahingehend problematisch, als es zwar einen höheren Siedepunkt als MeCl aufweist, mit diesem jedoch ein leicht siedendes Azeotrop bildet.

Ein wesentlicher Nachteil der Entfernung von niedrig und leicht siedenden Bestandteilen aus Methylchlorid durch Kondensation besteht darin, daß bei diesem Verfahren ein Abgas erhalten wird, das neben den genannten niedrig oder leicht siedenden Verunreinigungen auch nennenswerte Anteile an Methylchlorid enthält.

Es sind bereits Verfahren bekannt geworden, um das Methylchlorid zurückzugewinnen und von den unerwünschten Bestandteilen zu trennen.

So besteht z.B. ein Verfahren darin, den aus dem Kondensationssystem stammenden Gasstrom weiter zu komprimieren und z.B. mit Fluorkohlenwasserstoffen weiter abzukühlen. Hierbei werden neben Methylchlorid aber auch wieder nennenswerte Anteile der Kohlenwasserstoffe kondensiert.

Zur Rückgewinnung von Methylchlorid aus diesem Abgas wird in der SU-B 141 153 vorgeschlagen, das Abgas mit einem Absorptionsmittel für Methylchlorid zu waschen. Nachteil dieses Verfahrens ist, daß bei der Desorption das Methylchlorid durch das Absorptionsmittel verunreinigt ist.

In der DE-A 3 610 706 ist ein Verfahren beschrieben, bei dem die methylchloridhaltigen Abgase der Kondensation mit polaren Lösungsmitteln in Kontakt gebracht werden und die Verunreinigungen vom Methylchlorid durch eine selektive Abgaswäsche oder eine Extraktivdestillation abgetrennt werden. Bei Durchführung des Verfahrens werden hohe Rückgewinnungsraten an Methylchlorid aus dem Abgas erreicht, wobei das Methylchlorid in hoher Reinheit erhalten wird. Dieses Verfahren ist jedoch vergleichsweise aufwendig.

Aufgabe der Erfindung war daher ein Verfahren zur Reinigung von mit leicht oder niedrig siedenden C₁-C₃-Kohlenwasserstoffen und/oder iso-Butan verunreinigtem Methylchlorid bereitzustellen, welches eine wirksame Ausschleusung der das Methylchlorid verunreinigenden Kohlenwasserstoffe erlaubt und gleichzeitig die Methylchloridverluste minimiert.

Die Aufgabe konnte dadurch gelöst werden, daß das mit C₁-C₃-Kohlenwasserstoffen und/oder iso-Butan verunreinigte Methylchlorid destilliert und anschließend mehrstufig kondensiert wird, wobei die Kondensate als Rücklauf in die Destillationskolonne (10) zurückgeführt werden.

Mit diesem Verfahren kann auch Methylchlorid, wie es technisch üblicherweise durch Chlorierung von Methan oder durch Umsetzung von Methanol mit HCl erhalten wird, von Verunreinigungen befreit werden.

Gegenstand der vorliegenden Erfindung ist somit ein Verfahren zur Herstellung von Methylchlorsilanen durch Umsetzung von Silizium mit Methylchlorid in Anwesenheit von Katalysatoren und ggf. Promotoren, das die Schritte umfasst: Abtrennung der entstandenen Methylchlorsilane in einer Destillationskolonne; Zuführen des in der genannten Destillationskolonne abgetrennten Gasgemisches, das Methylchlorid, C₁-C₃-Kohlenwasserstoffe und Iso-Butan enthält, in eine Destillationskolonne, welches dadurch gekennzeichnet ist, dass der aus der Destillationskolonne entnommene Brüdenstrom mehrstufig kondensiert wird und mindestens ein Kondensat als Rücklauf in die Destillationskolonne zurückgeführt wird.

In einer bevorzugten Ausführungsform des erfindungsgemäßen Verfahrens wird eine 2- bis 5-stufige, besonders bevorzugt eine 3-stufige Kondensation durchgeführt.

Die Austrittstemperaturen liegen dabei vorzugsweise in einem Bereich von -70 bis 40°C. Bei der bevorzugten dreistufigen Kondensation weist die erste Stufe eine Austrittstemperatur von 10 bis 40°C, die zweite Stufe -20 bis 10°C und die dritte Stufe eine Austrittstemperatur von -70 bis -20°C auf.

Bei einer 2-stufigen Kondensation entfällt die bei der 3-stufigen Kondensation als Stufe 2 ausgewiesene Kondensation. Die Austrittstemperaturen liegen dann vorzugsweise in der ersten Stufe bei 25 bis 35°C, die der zweiten Stufe bei -30 bis -40°C.

Bei einer 4-stufigen Kondensation wird vorzugsweise eine zusätzliche Kondensationsstufe zwischen Stufe 2 und 3 der 3-stufigen Kondensation eingeschoben. Die Austrittstemperaturen liegen dann vorzugsweise folgendermaßen:

| | |
|---|---|
| Stufe 1 | 25 bis 35°C |
| Stufe 2 | -5 bis -15°C |
| Stufe 3 | -20 bis -30°C |
| Stufe 4 | -30 bis -40°C |

Bei einer 5-stufigen Kondensation werden vorzugsweise zwei zusätzliche Kondensationsstufen zwischen Stufe 1 und 2 und Stufe 2 und 3 der 3-stufigen Kondensation eingeschoben. Die Austrittstemperaturen liegen dann vorzugsweise folgendermaßen:

| | |
|---|---|
| Stufe 1 | 25 bis 35°C |
| Stufe 2 | 10 bis -10°C |
| Stufe 3 | -5 bis -15°C |
| Stufe 4 | -20 bis -30°C |
| Stufe 5 | -30 bis -40°C |

Für die erfindungsgemäße Destillation kann sowohl eine Packungskolonne als auch eine Bodenkolonne oder eine Kombination aus Packungs- und Bodenkolonne eingesetzt werden. Die Anzahl der theoretischen Stufen der Destillationskolonne beträgt vorzugsweise 2 bis 50, besonders bevorzugt 10 bis 20 theoretische Stufen.

In einer bevorzugten Ausführungsform des erfindungsgemäßen Verfahrens wird mindestens ein Kondensat in die Destillationskolonne zurückgeführt.

Die bei der mehrstufigen Kondensation anfallenden Kondensate werden vorzugsweise entweder gemeinsam oder getrennt am Kopf in die Destillationskolonne gegeben, wobei es vorteilhaft ist, den Zugabeort der kälteren Kondensate oberhalb der wärmeren Kondensate zu legen.

Die erfindungsgemäße Destillation kann bei einem Druck von 1 bis 60 bar betrieben werden. Vorzugsweise wird in der Destillationskolonne ein Druck von 5 bis 20 bar eingestellt.

Die Temperaturen der einzelnen Kondensationsstufen müssen den gewählten Betriebsdrücken angepaßt werden.

Bei der Durchführung des erfindungsgemäßen Verfahrens wird ein Methylchlorid sehr hoher Reinheit erhalten, das hervorragend als Einsatzstoff für die Herstellung von Methylchlorsilanen geeignet ist. Das bei der Destillation anfallende Abgas enthält nur noch geringe Anteile an Methylchlorid, wodurch Methylchloridverluste stark minimiert werden.

Der Ablauf des erfindungsgemäßen Verfahrens wird nachfolgend anhand der Reinigung von aus der Herstellung von Methylchlorsilanen stammenden Methylchlorid näher erläutert. Es handelt sich hierbei um eine besonders bevorzugte Variante des erfindungsgemäßen Verfahrens.

Das bei der Umsetzung von Silicium mit Methylchlorid in Anwesenheit von Katalysatoren und gegebenenfalls Promotoren entstehende feststoffhaltige Gasgemisch wird in einem Feststoffabscheider durch Gasfiltration oder durch Wäsche mit einem geeigneten Waschmedium von den siliciumhaltigen Feststoffen befreit. In einer Destillationskolonne werden zunächst die Methylchlorsilane abgetrennt, das anfallende Methylchlorid einer weiteren Destillation zugeführt, wo leichtsiedende Verunreinigungen, wie Methan, Ethan, Ethen, Propan, Propen, Wasserstoff und Sauerstoff, aus dem Methylchlorid abgetrennt werden.

Zu dieser Destillation wird vorzugsweise auch weiteres, nicht aus der Methylchlorsilansynthese stammendes ("frisches") Methylchlorid gegeben, um Restgehalte an leichtsiedenden Verunreinigungen auch aus diesem Einsatzstoff zu entfernen.

Die Zugabe des "frischen" Methylchlorids kann selbstverständlich auch an anderen Stellen, wie beispielsweise vor der destillativen Abtrennung der Methylchlorsilane, erfolgen. Das so gereinigte Methylchlorid wird dann vorzugsweise dem Methylchlorsilansynthesereaktor zugeführt.

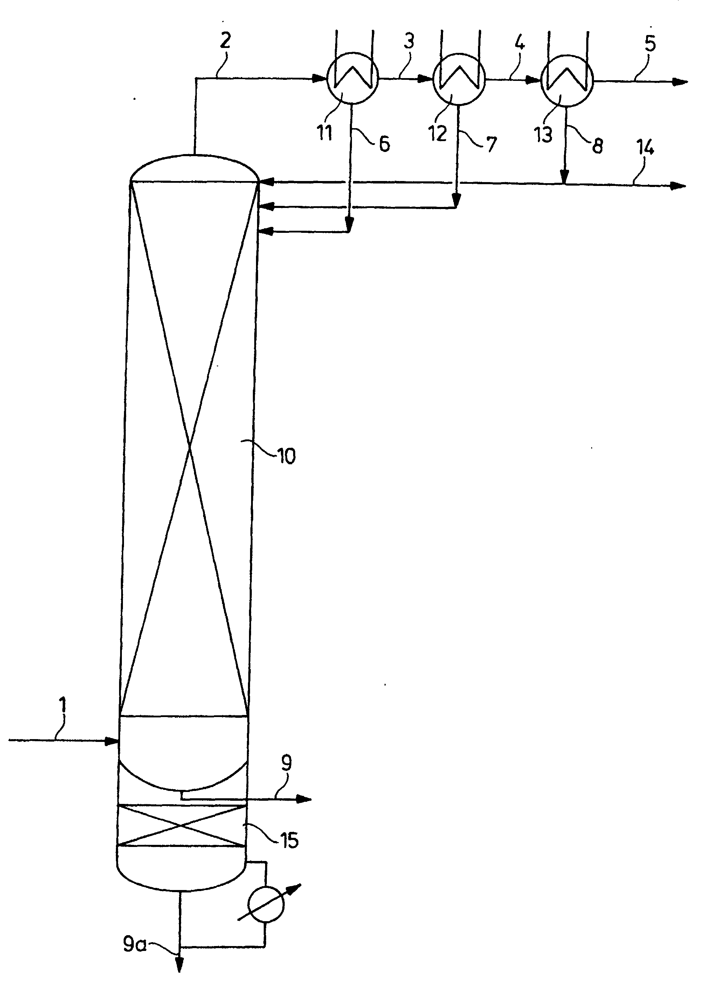

Das erfindungsgemäßen Verfahren wird in einer bevorzugten Ausführungsform bei 3-facher Kondensation unter Zuhilfenahme der Fig. 1 näher erläutert.

Das Verfahren wird dabei vorzugweise bei einem Druck zwischen 1 und 60 bar (absolut), besonders bevorzugt zwischen 5 und 20 bar durchgeführt.

Der verunreinigte Methylchloridstrom (1) tritt, vorzugsweise dampfförmig, d.h. so wie er üblicherweise im Methylchlorsilanproduktionsprozeß nach der Methylchlorsilanabtrennung anfällt, in den unteren Teil einer Destillationskolonne (10) ein, die vorzugsweise als reine Verstärkersäule ohne Energiezufuhr ausgeführt ist. Der Einsatz einer Abtriebssäule (15), mit der eine weitergehende Abreicherung der Verunreinigungen erreicht wird, ist jedoch nicht ausgeschlossen, allerdings muß dann zusätzlich Energie in einem Sumpfverdampfer aufgewandt werden. Die Kolonne kann mit üblichen Destillationsböden als Trennelement ausgestattet sein. Es können jedoch auch alle anderen technisch üblichen Einbauten für Stoffaustauschprozesse verwendet werden, wie z.B. Füllkörper oder geordnete Packungen. Am Kopf der Kolonne tritt der Brüdenstrom (2) aus, der in der ersten Kondensationsstufe (11) mit einem Kühlmittel mit einer Temperatur von 10 bis 40°C, vorzugsweise Luft oder Wasser, so weit wie möglich auskondensiert wird. Wird das erfindungsgemäße Verfahren bei weniger als 5 bar durchgeführt, so kann diese Kondensationsstufe entfallen. Der dabei anfallende flüssige Strom (6) wird vorzugsweise in den oberen Teil der Destillationskolonne (10) als Rücklauf aufgegeben. Das Restgas aus der ersten Kondensationsstufe (3) wird mit einem kälteren Kühlmittel als in der ersten Kondensationsstufe (11), z.B. Sole von -20°C oder flüssigem Ammoniak, in der 2. Kondensationsstufe (12) weiter abgekühlt, wobei der dabei anfallende flüssige Strom (7) ebenfalls als Rücklauf auf die Kolonne (10) aufgegeben wird. Der Rücklauf erfolgt jedoch etwas höher als der Rücklauf aus der ersten Stufe. Analog verfährt man in der dritten Kondensationsstufe (13), die jedoch bei noch tieferen Temperaturen als in der zweiten Stufe (12) betrieben wird, z.B. mit Sole von -45°C als Kühlmittel. In der 3. Kondensationsstufe (13) wird der Strom (8) auskondensiert und als Rücklauf auf den Kopf der Destillationskolonne (10) zurückgeführt. Im Restgasstrom (5) hinter der 3. Kondensationsstufe (13) liegen die gesättigten und/oder ungesättigten Kohlenwasserstoffe in konzentrierter Form vor, Methylchlorid ist nur noch in geringen Mengen enthalten. Im Sumpf der Kolonne wird mit dem Flüssigkeitsstrom (9) oder (9a) gereinigtes Methylchlorid entnommen. Strom (8) oder Teile davon können flüssig als Destillat (14) entnommen werden. Bevorzugt ist jedoch der Betrieb ohne Entnahme von Destillat (14).

Das beschriebene Verfahren kann dadurch vereinfacht werden, daß die Rücklaufströme 6, 7 und 8 gesammelt auf den Kolonnenkopf der Destillationskolonne (10) als Rücklauf aufgegeben werden.

Besonders bevorzugt ist die beschriebene Verfahrensweise ohne zusätzlichen Abtriebsteil (15). In diesem Fall wird keine Energie in Form von Heizdampf oder anderen Energieträgern zum Beheizen eines Sumpfverdampfers aufgebracht. Es fallen nur geringfügige Energiekosten für die Erzeugung der Kühlmittelströme für die 2. und 3. Kondensationsstufe (12) und (13) an.

Gegenstand der Erfindung ist zudem die Verwendung des nach dem erfindungsgemäßen Verfahren gereinigten Methylchlorids bei der Herstellung von Alkylhalogensilanen, vorzugsweise Methylchlorsilanen, durch die Umsetzung von Silicium mit Methylchlorid.

Die Herstellung von Methylchlorsilanen kann dabei z.B. nach den in US-A 2 380 991 und Wiss. Z. Techn. Univ. Dresden, 1633-1642 (1963) beschriebenen Verfahren erfolgen.

Das erfindungsgemäße Verfahren soll anhand des folgenden Beispiels näher erläutert werden, ohne daß dieses limitierend wirkt.

### Ausführungsbeispiel:

Das erfindungsgemäße Verfahren wurde in der in Fig. 1 abgebildeten Apparatur durchgeführt.

Das eingesetzte Methylchlorid 1 war folgendermaßen verunreinigt:
0,25 % Summe Methan, C₂-Kohlenwasserstoff
0,13 % Summe Stickstoff, Sauerstoff
0,02 % Argon
0,04 % CO₂
3,47 % iso-Butan
0,07 % Propan und
0,07 % Propen

100 Teile dieses Stroms wurden der in Fig. 1 abgebildeten Apparatur gasförmig zugeführt. Die Kolonne war als reine Verstärkersäule ausgeführt und war mit 38 Glockenböden ausgestattet. Das Kondensationssystem war dreistufig ausgeführt. Der Kopfdruck betrug 12 bar, der Brüdenstrom (2) verließ die Kolonne (10) mit 46,7°C. Die Kühlmittel für den Betrieb der einzelnen Kondensationsstufen wurden so gewählt, daß sich im Produktstrom (3) eine Temperatur von 30°C, im Produktstrom (4) von -15°C und im Produktstrom (5) von -30°C einstellte. Die Kolonne (10) wurde ohne flüssige Destillatentnahme (14) betrieben.

Am Sumpf der Kolonne fiel der Methylchloridstrom (9) bei einer Temperatur von 52°C mit folgenden Verunreinigungen an:
0,02 % Summe Methan
0,01 % Summe Stickstoff, Sauerstoff
0,01 % CO₂
3,43 % iso-Butan
0,04 % Propan und
0,04 % Propen.

Die Abreicherungen vom Strom (1) zum Strom (9) waren größtenteils ganz erheblich, ohne daß Energie in Form von Heizdampf aufgewandt werden mußte.

Produktstrom (5) enthielt 0,1 % des eingesetzten Methylchlorids, d.h. die Destillationsausbeute bezogen auf das Methylchlorid betrug 99,9 %.

## Patentansprüche

1. Verfahren zur Herstellung von Methylchlorsilanen durch Umsetzung von Silizium mit Methylchlorid in Anwesenheit von Katalysatoren und ggfs. Promotoren, das die Schritte umfasst:
- Abtrennung der entstandenen Methylchlorsilane in einer Destillationskolonne,
- Zuführen des in der genannten Destillationskolonne abgetrennten Gasgemisches, das Methylchlorid, C₁-C₃-Kohlenwasserstoffe und Iso-Butan enthält, in eine Destillationskolonne (10), **dadurch gekennzeichnet, daß** der aus der Destillationskolonne (10) entnommene Brüdenstrom (2) mehrstufig kondensiert wird und mindestens ein Kondensat als Rücklauf in die Destillationskolonne (10) zurückgeführt wird.

2. Verfahren nach Anspruch 1, **dadurch gekennzeichnet, daß** eine 2- bis 5-stufige Kondensation durchgeführt wird.

3. Verfahren nach einem der Ansprüche 1 oder 2, **dadurch gekennzeichnet, daß** die Kondensate in die Destillationskolonne (10) zurückgeführt werden.

4. Verfahren nach einem oder mehreren der Ansprüche 1 bis 3, **dadurch gekennzeichnet, daß** die Kondensation in drei Stufen durchgeführt wird, wobei die erste Stufe bei Austrittstemperaturen von 10 bis 40° C, die zweite Stufe bei -20 bis 10° C und die dritte Stufe bei -70 bis -20 C° betrieben wird.

5. Verfahren nach einem oder mehreren der Ansprüche 1 bis 4, **dadurch gekennzeichnet, daß** das verunreinigte Methylchlorid in einer Destillationskolonne (10) destilliert wird, die 2 bis 50 theoretische Stufen aufweist.

6. Verfahren nach einem der Ansprüche 1 bis 5, **dadurch gekennzeichnet, daß** der Druck bei der Destillation in der Destillationskolonne (10) 1 bis 60 bar beträgt.

7. Verfahren nach einem oder mehreren der Ansprüche 1 bis 6, **dadurch gekennzeichnet, daß** der Strom (8) oder Teile davon flüssig als Destillat (14) entnommen werden.

8. Verfahren nach einem oder mehreren der Ansprüche 1 bis 7, **dadurch gekennzeichnet, daß** das aus der Destillationskolonne (10) resultierende gereinigte Methylchlorid (9) in die Methylchlorsilansynthese zurückgeführt wird und die C₁-C₃-Kohlenwasserstoffe und/oder Isobutan als Restgasstrom (5) abgetrennt werden.

## Claims

1. Process for preparing methylchlorosilanes by reacting silicon with methyl chloride in the presence of catalysts and, if desired, promoters, which comprises the steps:
- separating off the resulting methylchlorosilane in a distillation column,
- feeding the gas mixture comprising methyl chloride, C₁-C₃-hydrocarbons and isobutane separated off in this distillation column into a distillation column (10), **characterized in that** the vapour stream (2) taken from the distillation column (10) is condensed in a plurality of stages and at least one condensate is returned as runback to the distillation column (10).

2. Process according to Claim 1, **characterized in that** a 2- to 5-stage condensation is carried out.

3. Process according to Claim 1 or 2, **characterized in that** the condensates are returned to the distillation column (10).

4. Process according to one or more of Claims 1 to 3, **characterized in that** the condensation is carried out in three stages, with the first stage being operated at outlet temperatures of from 10 to 40°C, the second stage being operated at from -20 to 10°C and the third stage being operated at from -70 to -20°C.

5. Process according to one or more of Claims 1 to 4, **characterized in that** the contaminated methyl chloride is distilled in a distillation column (10) having from 2 to 50 theoretical plates.

6. Process according to one or more of Claims 1 to 5, **characterized in that** the pressure in the distillation in the distillation column (10) is from 1 to 60 bar.

7. Process according to one or more of Claims 1 to 6, **characterized in that** the stream (8) or part thereof is taken off in liquid form as distillate (14).

8. Process according to one or more of Claims 1 to 7, **characterized in that** the purified methyl chloride (9) resulting from the distillation column (10) is returned to the methylchlorosilane synthesis and the C₁-C₃-hydrocarbons and/or isobutane are separated off as tailgas stream (5).

## Revendications

1. Procédé destiné à la fabrication de méthylchlorosilanes par la réaction de silice avec du chlorure de méthyle en présence de catalyseurs et, le cas échéant, d'activateurs, qui comprend les étapes consistant à :
séparer le méthylchlorosilane formé dans une colonne de distillation,
amener le mélange gazeux séparé dans ladite colonne de distillation, lequel mélange contient le chlorure de méthyle, des hydrocarbures en C₁ à C₃ et de l'isobutane, dans une colonne de distillation (10), **caractérisé en ce que** le flux de vapeur (2) prélevé de la colonne de distillation (10) se condense en plusieurs étapes et **en ce qu'**au moins un condensat est renvoyé dans la colonne de distillation (10) en tant que reflux.

2. Procédé selon la revendication 1, **caractérisé en ce qu'**une condensation est réalisée en 2 à 5 étapes.

3. Procédé selon l'une des revendications 1 ou 2, **caractérisé en ce que** le condensat est renvoyé dans la colonne de distillation (10).

4. Procédé selon l'une ou plusieurs des revendications 1 à 3, **caractérisé en ce que** la condensation s'effectue en trois étapes, moyennant quoi la première étape est menée à des températures de sortie de 10 à 40°C, la deuxième étape à des températures de -20 à 10 C et la troisième étape à des températures de -70 à -20°C.

5. Procédé selon l'une ou plusieurs des revendications 1 à 4, **caractérisé en ce que** le chlorure de méthyle contaminé est distillé dans une colonne de distillation (10) qui présente 2 à 50 étapes théoriques.

6. Procédé selon l'une quelconque des revendications 1 à 5, **caractérisé en ce que** la pression s'élève à une valeur entre 1 et 60 bars lors de la distillation dans la colonne de distillation (10).

7. Procédé selon l'une ou plusieurs des revendications 1 à 6, **caractérisé en ce que** le flux (8) ou des parties de ce dernier sont prélevés sous forme liquide en tant que distillat (14).

8. Procédé selon l'une ou plusieurs des revendications 1 à 7, **caractérisé en ce que** le chlorure de méthyle purifié obtenu (9) de la colonne de distillation (10) est renvoyé dans la synthèse de méthylchlorosilane et **en ce que** les hydrocarbures en C₁ à C₃ et/ou l'isobutane est(sont) séparé(s) sous forme de flux de gaz résiduaire (5).
